Europäisches Patentamt

European Patent Office

Office européen des brevets

(11) Veröffentlichungsnummer: **0 256 158**
**A1**

## (12) EUROPÄISCHE PATENTANMELDUNG

(21) Anmeldenummer: **86111347.0**

(22) Anmeldetag: **16.08.86**

(51) Int. Cl.⁴: **A61F 2/80**

(43) Veröffentlichungstag der Anmeldung:
**24.02.88 Patentblatt 88/08**

(84) Benannte Vertragsstaaten:
**AT BE CH DE FR GB IT LI LU NL SE**

(71) Anmelder: **Ipos Gesellschaft für integrierte Prothesen-Entwicklung und orthopädietechnischen Service mbH & Co. KG**
**Lüner Rennbahn 14**
**D-2120 Lüneburg(DE)**

(72) Erfinder: **Prahl, Jan**
**Nutzfelder Weg 13**
**D-2127 Rullstorf(DE)**

(74) Vertreter: **Patentanwälte Dipl.-Ing. J. Richter**
**Dipl.-Ing. F. Werdermann**
**Neuer Wall 10**
**D-2000 Hamburg 36(DE)**

(54) **Prothese, wie Oberschenkel-Prothese u.dgl., sowie Vorrichtung und Verfahren zur Herstellung von Prothesen.**

(57) Bei einer Prothese, die unter Verwendung eines Negativmodells hergestellt wird, wird das Negativmodell durch Formen des entsprechenden Patienten-Körperteiles erzeugt und weist eine längliche Grundform auf, die mindestens an einem Ende offen ist und deren Innenform der Innenfläche des Körper-Anschlußstücks der herzustellenden Prothese entspricht. Bei dem Negativmodell ist eine je einem Ende zugeordnete Führungskappe (14,16) vorgesehen, die an dem ihr zugeordneten Ende befestigt ist und die je eine Lagervorrichtung (18,20) für eine Führungseinrichtung (12) aufweist, die sich von einer Führungskappe zu derjenigen am gegenüberliegenden Ende erstreckt. Eine Abtastvorrichtung (22) ist im Innenraum des Negativmodells verschiebbar und um die Mittelachse der Führungseinrichtung (12) drehbar gelagert und gibt Meßdaten über das Profil des Negativmodells (10) ab, wobei die Prothese unter Verwendung dieser beim Prothesen-Hersteller gespeicherten Meßdaten hergestellt wird, so daß zur Versorgung eins Patienten das von diesem abgenommene und für die Prothesenherstellung erforderliche Negativmodell nicht mehr dem Prothesen-Hersteller zugesandt werden muß.

FIG.1

## Prothese, wie Oberschenkel-Prothese u.dgl., sowie Vorrichtung und Verfahren zur Herstellung von Prothesen.

Die Erfindung betrifft eine Prothese, wie Oberschenkel-Prothese u.dgl., die unter Verwendung eines Negativmodells hergestellt wird, wobei das Negativmodell durch Abformen des entsprechenden Patienten-Körperteiles erzeugt wird und eine längliche Grundform aufweist, die mindestens an einem Ende offen ausgebildet ist und deren Innenform der Innenfläche des Körper-Anschlußstückes der herzustellenden Prothese entspricht, sowie ferner eine Vorrichtung und ein Verfahren zur Herstellung einer Prothese, wie Oberschenkel-Prothese u.dgl. , bei welchem ein Negativmodell unter Abformung von Restkörperteilen eines Patienten erstellt wird, wobei eine erhärtende Masse, insbesondere Gips, in verformbarem Zustand auf das Restkörperteil in einer Länge aufgebracht wird, die dem beabsichtigten Anlage-und Abstützbereich der Prothese an dem Restkörperteil entspricht, wobei das Negativmodell nach dem Erhärten in einer im wesentlichen kuppenförmigen Grundform von dem Restkörperteil abgenommen wird.

Bekannte Prothesen werden dadurch hergestellt, daß der für den Anschluß an die Prothese bestimmte Körperteilrest des Patienten mit einer Gipsschicht versehen wird. Nach Erhärten wird die Gipsschicht abgenommen und dient als Negativmodell für die Herstellung des Körper-Anschlußstückes der Prothese. Zur eigentlichen Herstellung der Prothese wird entweder ein Positivmodell durch Ausgießen des Negativmodells erzeugt,das für die Erzeugung des Anschlußstückes der Prothese verwendet wird, oder das Anschlußstück wird von Hand hergestellt,wobei das Negativmodell als Vorlage dient.

Beide Arten der Herstellung sind jedoch nachteilig weil zum einen die Verwendung eines zusätzlichen Positivmodells zusätzliche Fehlermöglichkeiten mit sich bringt, zum anderen die handwerkliche Arbeit sehr exakt durchgeführt werden muß, wenn ein hoher Tragekomfort erzielt werden soll.

Ein weiterer Nachteil der bekannten Verfahren bzw. Prothesen besteht darin, daß eine Änderung des Anschlußstücks der Prothese, wie sie durch die Belastungsveränderungen, die für den Patienten entstehen, erforderlich werden können, nur - schwer durchgeführt werden kann. Entweder muß ein komplett neues Anschlußstück erstellt werden, oder aber es erfolgt eine nachträgliche Anpassung, die jedoch in ihrer Qualität an die ursprüngliche Anpassung nicht heranreicht.

Die Versorgung von Patienten mit Prothesen ist oftmals sehr zeitaufwendig, insbesondere dann, wenn die Patienten in abgelegenen Orten oder Gegenden versorgt werden sollen, in denen selbst keine Versorgung vorgenommen werden kann und wenn, dann oftmals nur mit primitiven Prothesen oder Prothesen-Hilfen, wenn hunderte von Kilometern entfernt z.B. von einer Landeshauptstadt Versorgungen von Patienten mit Prothesen vorgenommen werden sollen. Die Abnahme der für die herzustellenden Prothesen erforderlichen Maße läßt sich vor Ort häufig nicht durchführen, da die hierzu erforderlichen Einrichtungen fehlen. Werden Negativmodelle angefertigt, dann müssen diese an den Prothesen-Hersteller gesandt werden, der wiederum nach der Herstellung der Prothese diese an den Patienten senden muß. Auf diese Wise wird viel Zeit benötigt; außerdem entstehen hohe Transport kosten. In vielen Fällen muß der zu versorgende Patient u.a. auch lange Transportwege zurücklegen, damit die für die Anfertigung der Prothese erforderlichen Maße abgenommen oder ein Negativmodell angefertigt werden kann.

Die Erfindung löst die Aufgabe, eine Prothese , eine Vorrichtung und ein Verfahren gemäß der eingangs beschriebenen Art zu schaffen, bei welchem ein kostengünstiger, schneller und fehlertoleranter Weg zur Herstellung der Prothese ermöglicht wird, ohne daß der Patient lange Transportwege zurücklegen muß, um eine Prothese zu erhalten.

Zur Lösung dieser Aufgabe wird eine Prothese, wie Oberschenkel-Prothese u.dgl. gemäß der eingangs beschriebenen Art vorgeschlagen, die erfindungsgemäß in der Weise ausgebildet ist, daß eine je einem Ende zugeordnete Führungskappe an dem Negativmodell vorgesehen ist, die an dem ihr zugeordneten Ende befestigt ist und die je eine Lagervorrichtung für eine Führungseinrichtung aufweist, die sich von einer Führungskappe zu derjenigen am gegenüberliegenden Ende erstreckt, und daß eine Abtastvorrichtung verschiebbar und um die Mittelachse der Führungseinrichtung drehbar gelagert ist und Meßdaten über das Profil des Negativmodells abgibt, wobei die Prothese unter Verwendung dieser Meßdaten hergestellt wird.

Die Erfindung sieht ferner eine Vorrichtung zur Herstellung einer Prothese, wie Oberschenkel-Prothese u.dgl., vor, bei der ein Negativmodell unter Abformung von Restkörperteilen eines Patienten erstellt wird, wobei eine erhärtende Masse, insbesondere Gips, in verformbarem Zustand auf das Restkörperteil in einer Länge aufgebracht wird, die dem beabsichtigten Anlage-und Abstützbereich der Prothese an dem Restkörperteil entspricht, wobei das Negativmodell nach dem Erhärten in einer wesentlichen kuppenförmigen Grundform von dem Restkörperteil abgenommen wird und mit der das Vermessen des hergestellten Negativmodells in einfachster Weise und kostensparend möglich ist. Hierzu besteht die Vorrichtung aus einem Geräteteil mit in dessen Innenraum angeordneter Laserstrahlerzeugungseinrichtung und mit einer auf dessen oberer Abdeckplatte angeordneten, ringförmigen Manschette aus einem federnd-elastischen Werkstoff, wie z.B. Gummi oder gummielastischem Kunststoff, zur Aufnahme und Halterung eines Negativmodells, wobei das Geräteteil einen ein-und ausfahrbaren und in dem Innenraum des Negativmodells einfahrbaren Abtastkopf einer Abtasteinrichtung aufweist, der in Längs-und Umfangsrichtung bewegbar ist und der Laserstrahlen in einem Winkel von etwa 45° aussendet, deren Reflexionssignale an der Innenfläche des Negativmodells erfaßbar sind und wobei ein Analog/Digital-Wandler vorgesehen ist, der ansprechend auf das insbesondere verstärkte Ausgangssignal des Abtastkopfes dem Innenprofil des Negativmodells entsprechende Meß-Daten abgibt.

Die Aufgabe wird ferner durch ein Verfahren gemäß der eingangs beschriebenen Art in der Weise gelöst, daß erfindungsgemäß die Spitze des Negativmodells mit der kuppenförmigen Grundform abgeschnitten wird, so daß das Negativmodell eine im wesentlichen röhrenförmige Grundform erhält, daß auf jedes Ende der röhrenförmigen Grundform des Negativmodells eine Führungskappe aufgesetzt wird, die eine Lagervorrichtung aufweist, in welcher eine Führungseinrichtung gelagert ist, die sich zwischen den gegenüberliegenden Führungskappen erstreckt, daß die Führungskappen beim Aufsetzen auf die offenen Enden des Negativmodells zueinander so ausgerichtet werden, daß die Mittelachse ihrer Lagervorrichtungen zueinander im wesentlichen koaxial sind, daß auf der Führungseinrichtung eine Abtastvorrichtung in Längsrichtung und in Umfangsrichtung der Führungseinrichtung geführt wird und daß die Abtastvorrichtung das Innenprofil des Negativmodells abtastet und die so ermittelten Meßwerte für die Erzeugung der Prothese mittels einer automatischen Einrichtung verwendet werden.

Dadurch, daß die Innenprofilierung des Negativmodells unmittelbar abgetastet wird, können zunächst die Fehler vermieden werden, die durch das Zwischenschalten eines weiteren Verfahrensschrittes mittels des Positivmodells entstehen können. Erfindungsgemäß können die mit der erforderlichen Auflösung und Genauigkeit gewonnenen Meßdaten unmittelbar zur Steuerung einer Werkzeugmaschine verwendet werden, die das Anschlußstück für die Prothese herstellt.

Die Führungseinrichtung ist zweckmäßigerweise zumindest einseitig elastisch in der Führungskappe aufgehängt. Daraus ergibt sich der besondere Vorteil, daß Ausrichtfehler ausgeglichen werden können.

Ein weiterer besonderer Vorteil besteht darin, daß keine Zentrierung der Führungseinrichtung gegenüber der Längsachse des Negativmodells erforderlich ist. Es ist lediglich erforderlich, daß die Abtastvorrichtung über die gesamte Länge der Führungseinrichtung frei rotieren kann.

Es ist auch möglich, eine sich gerade erstreckende Führungseinrichtung bei leicht abgewinkelten Negativmodellen - etwa für Unterschenkelprothesen, deren Anschlußstück sich über das Knie erstreckt - zu verwenden.

Die Führungskappen können für eine Vielzahl unterschiedlicher Negativmodelle verwendet werden. Zweckmäßigerweise sind sie elastisch ausgebildet, so daß sie sowohl für Arm-als auch für Beinprothesen eingesetzt werden können. Besonders vorteilhaft ist es, daß die vergleichsweise teuere Abtastvorrichtung lediglich einfach erforderlich ist. Besonders vorteilhaft ist es ferner, wenn die Abtastvorrichtung radial vergleichsweise kurz gebaut und insbesondere etwa in der Mitte von der Führungseinrichtung durchtreten wird.

Die Befestigung der Abtastvorrichtung an der Führungseinrichtung ist lösbar. Es ist jedoch wichtig, daß während der Abtastung keine Relativbewegung zwischen der Abtastvorrichtung und der Führungseinrichtung auftritt, damit die erforderliche Genauigkeit eingehalten werden kann. Ferner muß die Führungseinrichtung gegenüber dem Negativmodell sicher gelagert sein. Dies kann durch die starre Ausbildung einer Führungskappe oder durch zusätzliche Abstützungen gewährleistet werden.

Vor der eigentlichen Abtastung wird das Negativmodell, sofern es einseitig geschlossen ist, an diesem Ende abgeschnitten. Eine Führungskappe wird mit ihrer Lagervorrichtung auf die Führungseinrichtung aufgeschraubt. Dann wird die Abtastvorrichtung auf die Führungseinrichtung aufgesteckt und etwa in der Mitte der Führungseinrichtung befestigt. Das Negativmodell wird auf die Führungseinrichtung auf gesteckt, und in die bereits gelagerte Führungskappe wird das entsprechende Ende des Negativmodells gestülpt. Dann wird die andere Führungskappe mit ihrer Lagervorrichtung auf die Führungseinrichtung aufgeschraubt und ebenfalls über das Führungsmodell gestülpt. Vorteilhafterweise weisen die Führungskappen einen durchsichtigen Bereich auf, so daß die Ausrichtung der Abtastvorrichtung in dem Negativmodell kontrolliert werden kann und gegebenenfalls eine Anpassung vorgenommen werden kann, um sicherzustellen, daß die Abtastvorrichtung sich in dem Negativmodell in allen Richtungen frei bewegen kann.

Die Übermittlung der Meßdaten von der Abtastvorrichtung geschieht entweder drahtlos, wenn die Abtastvorrichtung batteriebetrieben ist, oder durch Anschlußkabel, die in der Führungseinrichtung vorgesehen sind.Gleiches gilt für die für die Spannungsversorgung der Abtastvorrichtung erforderlichen Kabel.

Es ist auch möglich, die Führungseinrichtung als Spindel aus einem elektrisch isolierenden Material auszubilden und entlang ihrer ganzen Länge je sich längs erstreckende Kontaktflächen aufzukaschieren, die zur Spannungsversorgung der Abtastvorrichtung und zur Übermittlung der Meßdaten verwendet werden können.

Eine weitere Möglichkeit besteht darin, die Führungseinrichtung zweiteilig auszubilden, so daß ein Teil von oben und ein weiterer Teil von unten in die Abtastvorrichtung eingesteckt wird. Diese beiden Teile könnten zugleich zur Spannungsversorgung verwendet werden.

Es ist ferner möglich, das Meßsignal als der Span nungszuleitung überlagertes Wechselstromsignal auszubilden und so lediglich mit zwei Anschlußleitungen sowohl für die Spannungsversorgung als auch für die Übermittlung der Meßdaten auszukommen.

Die Ausführungsform einer Vorrichtung mit in den Innenraum des Negativmodells einfahrbaren Laserstrahlen aussendendem Abtastkopf ist besonders kostengünstig und wirtschaftlich, da diese Vorrichtung in den medizinischen Versorgungsstationen, wie z.B. Ambulanzen, Ärzten, Krankenhäusern u.dgl., aufgestellt dem Arzt oder Orthopäden die Möglichkeit gibt, ein hergestelltes Negativmodell sofort nach dem Erhärten zu vermessen und die erhaltenen Meßwerte sogleich zur Herstellung der Prothese an eine Zentralstelle für eine Prothesenherstellung weiterzugeben, so daß der Versandweg des Negativmodells zum Prothesenhersteller entfällt. Beim Prothesenhersteller werden die eingegangenen Meßdaten zur Person des Patienten oder Prothesenträgers gespeichert und nach diesen Meßdaten wird die Prothese angefertigt. Veränderungen am Stumpf des Patienten werden dann der Zentralstelle mitgeteilt, die dann anhand der vorliegenden Meßdaten und der neu eingegangenen Werte eine neue Prothese herstellen kann, wenn die alte Prothese nicht mehr korrigiert werden kann, so daß in diesem Fall dann auch ohne ein neu angefertigtes Negativmodell ausgekommen werden kann.

Vorteilhafte Weiterbildungen der Erfindung ergeben sich aus den Unteransprüchen.

Im folgenden wird der Gegenstand der Erfindung anhand von Ausführungsbeispielen in den Zeichnungen erläutert. Es zeigt

Fig. 1 eine perspektivische Ansicht eines Negativmodells mit aufgesetzten Führungskappen und einer sich durch das Negativmodell hindurch erstreckenden Führungseinrichtung bei der Anwendung des erfindungsgemäßen Verfahrens,

Fig. 2 eine schematische Darstellung einer anderen Ausführungsform einer erfindungsgemäßen Führungsvorrichtung und Abtastvorrichtung und

Fig. 3 in einer schematischen Seitenansicht teils im Schnitt eine weitere Ausführungsform einer Vorrichtung zum Vermessen eines Negativmodells zur Herstellung einer Prothese.

Die Prothese - der Begriff "Prothese" schließe in diesem Zusammenhang auch Orthesen mit ein - wird unter Anwendung des Verfahrens hergestellt. Zunächst ist die Anfertigung eines Negativmodells erforderlich.

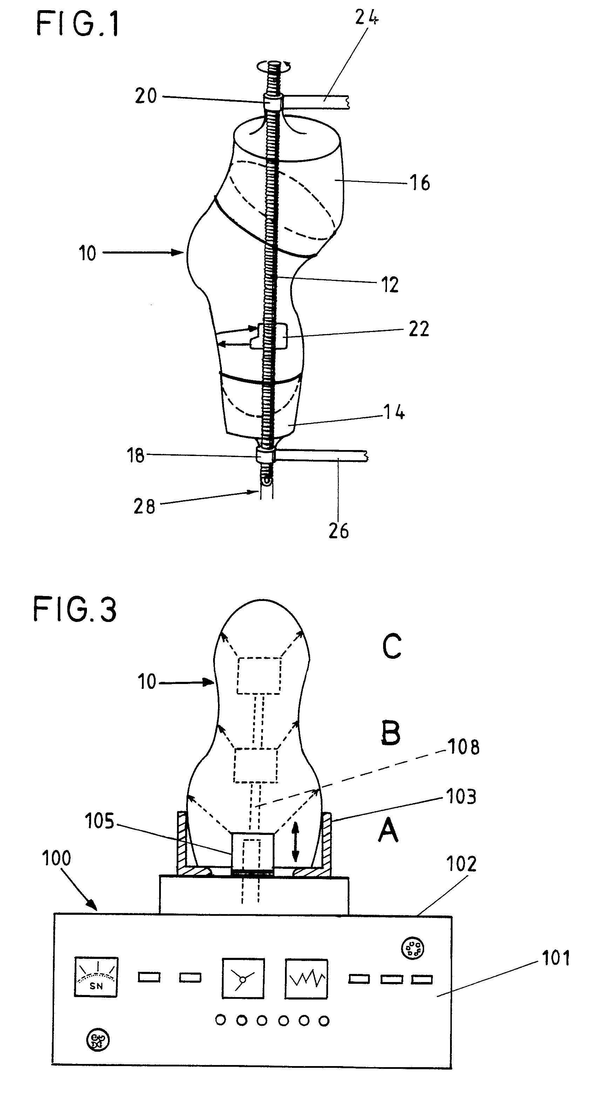

Das in Fig. 1 dargestellte Negativmodell 10 wurde dadurch gewonnen, daß ein Abdruck von einem Rest eines zu ersetzenden Körperteils geformt wurde. Ein derartiger Anwendungsfall tritt beispielsweise dann auf, wenn eine teilweise Amputation eines Unterschenkels vorliegt. Dabei erstreckt sich das Ngativmodell über das Knie des Patienten hinweg, um einen besseren Anschluß zu bilden. In an sich bekannter Weise wird ein Formsegment als Grundlage für die Erstellung des Negativmodells verwendet und mit Gips oder gipsähnlichen Materialien eine Feinanpassung vorgenommen.

Nach Erhärten des Gipses wird das Negativmodell vorsichtig abgelöst. Im Falle eines Negativmodells der in Fig. 1 dargestellten Form ist es erforderlich, das Negativmodell in Längsrichtung aufzuschneiden, um ein Ablösen zu ermöglichen.

Nach dem Ablösen wird das Negativmodell soweit wie möglich in die ursprüngliche Form gebracht. Das untere, geschlossene Ende wird abgeschnitten, und zwar zumindest soweit, daß eine Öffnung entsteht, die die Aufnahme einer Führungseinrichtung 12 ermöglicht.

Auf die beiden Enden des Negativmodells 10 werden nun zwei, je in Standardgrößen vorkonfektionierte Führungskappen 14 und 16 aufgesetzt. Die Führungskappen 14 und 16 bestehen zum großen Teil aus einem elastischen Material, beispielsweise Gummi, und sind so ausgelegt, daß sie über je ein Ende des Negativmodells 10 gestülpt werden können. In ihrer Mitte ist je eine metallische Lagervorrichtung 18 und 20 einvulkanisiert. Die Lagervorrichtungen 18 und 20 weisen je ein Innengewinde zur Aufnahme der als Spindel ausgebildeten Führungs einrichtung auf.

Die Montage der in Fig. 1 dargestellten Anordnung erfolgt dergestalt, daß zunächst die untere Führungskappe 14 auf die Führungseinrichtung 12 aufgeschraubt wird, dann die Abtastvorrichtung 22 auf die Führungseinrichtung 12 aufgesteckt und an geeigneter Stelle arretiert wird, dann das Negativmodell 10 über die Führungseinrichtung 12 geschoben wird und die untere Führungskappe 14 über das untere Ende des Negativmodells gestülpt wird, dann die obere Führungskappe 16 auf die Führungseinrichtung 12 aufgeschraubt wird und über das obere Ende des Negativmodells 10 gestülpt wird.

Nach der Montage wird überprüft, ob die Abtastvorrichtung 22 sich in dem Negativmodell frei drehen läßt. Erforderlichenfalls wird eine Anpassung über eine der elastischen Führungskappen 14 und 16 vorgenommen.

Die Lagervorrichtungen 18 und 20 sind neben der Abstützung an den Führungskappen 14 und 16 je an Trägern 24 und 26 abgestützt, die beide an einem nicht dargestellten Rahmen in geeigneter Weise gehalten sind.

Die Führungseinrichtung 12 ist ferner an ihrem unteren Ende mit einer in Fig. 1 nicht dargestellten Antriebsvorrichtung über einen Wellenstummel 28 verbunden.

Die Antriebsvorrichtung treibt die spindelförmige Führungseinrichtung 12 mit gleichmäßiger Geschwindigkeit an, so daß die Abtastvorrichtung 22 entlang eines spiralförmigen Weges das Negativmodell 10 von unten nach oben abtastet. Die Drehgeschwindigkeit beträgt dabei etwa eine Umdrehung pro Sekunde, wobei pro Umdrehung 360 Meßpunkte erfaßt werden. Die Steigung des Gewindes der Führungseinrichtung 12 beträgt etwa 2 mm. Es hat sich herausgestellt, daß die durch diese Daten erzielbare Auflösung vollkommen ausreichend ist, wobei ein Meßfehler von +/-0,5 mm bei der Abtastvorrichtüng 22 toleriert werden kann.

Die erfaßten Meßdaten entsprechen je dem Abstand eines jeden Meßpunktes von der Führungseinrichtung. Die Meßdaten werden einem Mikrocomputer zugeleitet, der die Meßdaten aufbereitet und auf einem Datenträger zwischenspeichert. Anstelle dessen können die Meßdaten auch über eine On-line-Verbindung einem Zentralrechner zugeleitet werden, der dann unmittelbar eine numerisch gesteuerte Werkzeugmaschine ansteuern kann, die das Anschlußstück für die neu zu erstellende Prothese aufgrund dieser Meßdaten herstellt.

Vorteilhafterweise werden die Meßdaten in der aufbereiteten Form zwischengespeichert, so daß, wenn beispielsweise der Umfang des Körperteilrestes des Patienten im laufe der Zeit abgenommen hat, auf einfache Weise eine Korrektur des Anschlußstücks der Prothese ermöglicht wird, ohne daß ein neues Negativmodell erstellt werden muß. In einer Zentralstelle können dann die Meßdaten einer großen Anzahl von Patienten bzw. Prothesenträgern, unabhängig davon, wo diese ihren Sitz haben, gespeichert werden.

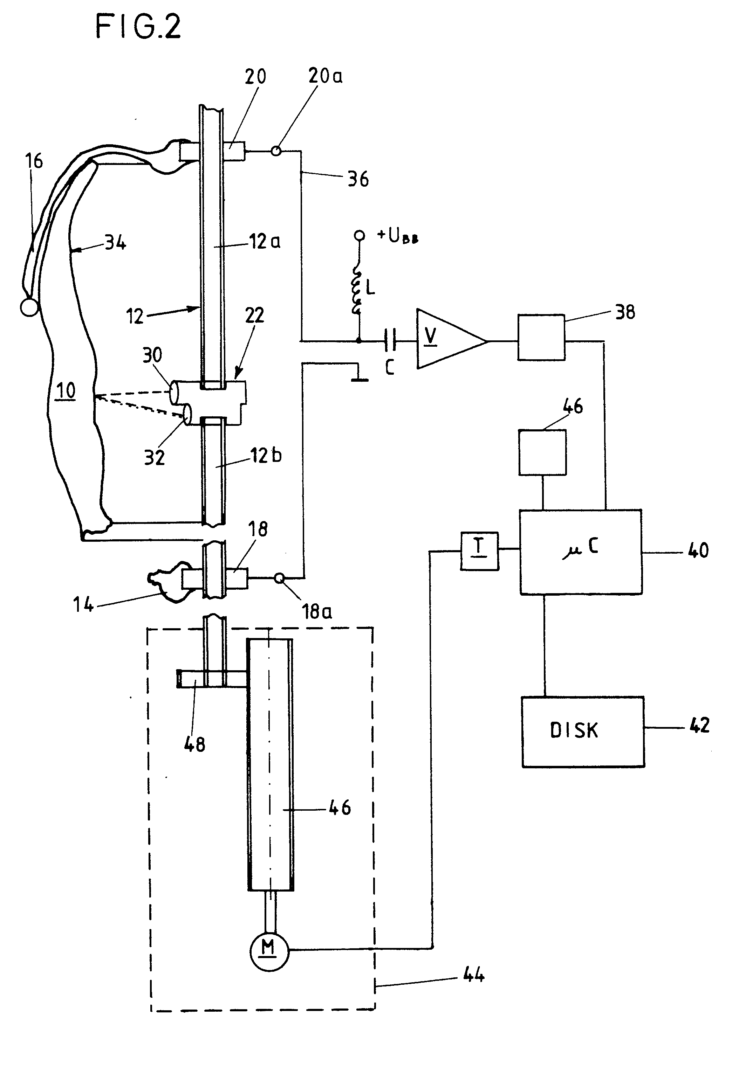

In Fig. 2 ist die Ansteuerung und Signalauswertung der Vorrichtung gemäß Fig. 1 im einzelnen dargestellt Gleiche Bezugszeichen weisen auf gleiche Teile hin. Die Abtastvorrichtung 22 weist eine Laser-Lichtquelle mit einer Sendeoptik 30 und eine Empfängervorrichtung mit einer Empfangsoptik 32 auf. In an sich bekannter Weise wird über die Abtastvorrichtung 22 die Entfernung der Innenfläche 34 des Negativmodells 10 von der Führungseinrichtung 12 gemessen. Die Führungseinrichtung 12 ist in der in Fig. 2 dargestellten Ausführungsform zweigeteilt. Sie wird von oben und von unten in die Abtastvorrichtung 22 eingesteckt und dient außerdem zur Spannungsversorgung und zur Rückleitung der Meßsignale. Die Spindeln 12a und 12b der Führungseinrichtung 12 bestehen daher aus leitfähigem Material. Ebenso sind die Lagervorrichtungen 20 und 18 aus Metall. Über einen Anschluß 18a ist die untere Lagereinrichtung mit Masse verbunden. Die obere Lagervorrichtung 20 ist über einen Anschluß 20a mit einer Leitung 36 verbunden. Die Leitung 36 wird über eine Spule L von einer Betriebsspannungsquelle $+U_{BB}$ mit Gleichspannung versorgt, die als Betriebsspannung für die Laserlichtquelle und für einen in der Abtastvorrichtung 22 vorgesehenen Vorverstärker der Empfangsvorrichtung dient. Das Ausgangssignal des Vorverstärkers, das als Wechselstromsignal aufbereitet ist, wird ebenfalls über die Leitung 36 und einen Kondensator C einem Verstärker V zugeleitet. Nach Gleichrichtung wird das Ausgangssignal des Verstärkers V einem A/D-Wandler 38 zugeleitet, der das an seinem Eingang anstehende Analogsignal in ein Digitalsignal umwandelt. Das Ausgangssignal des A/D-Wandlers 38 wird einem Mikrocomputer 40 zugeleitet, der aus den Meßdaten Formdaten berechnet und die Formdaten auf einem Hintergrundspeicher, beispielsweise einer Diskette 42, abspeichert. Ferner ist mit dem Mikrocomputer ein Treiber T verbunden, dessen Ausgangsanschluß mit einem Motor M verbunden ist. Der Motor M stellt einen Teil einer Antriebsvorrichtung 44 dar. Ferner ist an den Mikrocomputer 40 eine Bedieneinheit 46 angeschlossen, die zum Start des Motors 44 dient. Wenn der Abtastzyklus gestartet worden ist, erfolgt die weitere Steuerung durch den Mikrocomputer 40, der ein automatisches Ablaufen eines Abtastzyklus erlaubt.

Die Abtastvorrichtung 44 weist ferner ein mit dem Motor M drehfest verbundenes Zahnrad 46 auf, das mit einem mit der Führungseinrichtung 12 verbundenen Zahnrad 48 kämmt. Eines der Zahnräder 46 und 48 ist von einer Länge, die den Eingriff des anderen Zahnrades unabhängig von der Stellung der Führungseinrichtung gegenüber den Lagervorrichtungen 18 bzw. 20 erlaubt.

Zur Vermeidung von Brummschleifen ist es vorteilhaft, wenn das Zahnrad 46 aus einem elektrisch isolierenden Material besteht.

Die in Fig. 3 dargestellte Vorrichtung 100 dient ebenso wie die in Fig. 1 dargestellte und vorangehend beschriebene Anordnung zur Herstellung einer Prothese, d.h., sie dient zur Erfassung der Meßwerte für die Herstellung von Prothesen. Die Vorrichtung 100 besteht aus einem kastenförmigen Geräteteil 101, auf dessen oberer Abdeckplatte 102 eine ringförmige Manschette 103 aus federndelastischen Werkstoffen, wie z.B. Gummi oder gummielastischen Kunststoffen, angeordnet ist. Diese Manschette 103 dient zur Aufnahme und Halterung des auf das Geräteteil 101 aufgesetzten Negativmodells 10, das so in die Manschette 103 eingesetzt wird, daß die Öffnung des Negativmodells dem Geräteteil zugekehrt ist. Das Geräteteil 101 weist ferner einen in der Höhe verfahrbaren Abtastkopf 105 einer geeigneten und vorangehend beschriebenen Abtastvorrichtung auf, der bei Inbetriebnahme der Vorrichtung Laserstrahlen in einem Winkel von etwa 45° aussendet, wobei die Laserstrahlenerzeugungseinrichtung im Innenraum des Gehäuseteils 101 angeordnet ist. Die Höhenverfahrbarkeit des Abtastkopfes 105 wird mittels eines Teleskopstabes 108 erreicht,so wie dies in Fig. 3 angedeutet ist. Die Arbeitsweise der Vorrichtung 100 in Bezug auf die Abtastvorrichtung ist wie vorangehend beschrieben, ebenso der Erhalt der Meßwerte und deren Verarbeitung und Speicherung, um dann nach dem vermessenen Negativmodell die Prothese in an sich bekannter Weise herzustellen. Die kontinuierliche Verfahrbarkeit des Abtastkopfes 105 wird mittels einer in dem Geräteteil 101 angeordneten Antriebseinrichtung, wie Antriebsmotor od.dgl., vorgenommen. Neben der Höhenverfahrbarkeit kann der Abtastkopf 105 um eine senkrechte Achse verdrehbar sein. Eine Verfahrbarkeit des Abtastkopfes ist durch die Stellungen A,B und C angedeutet. Der Abtastkopf 105 umfaßt die Laser-Lichtquelle mit einer Sendeoptik und eine Empfängervorrichtung mit einer Empfangsoptik. Der Teleskopstab 108 dient gleichzeitig zur Aufnahme der Einrichtungen zur Spannungsversorgung und zur Rückleitung der Meßsignale.

**Ansprüche**

1. Prothese, die unter Verwendung eines Negativmodells hergestellt wird, wobei das Negativmodell durch Abformen des entsprechenden Patienten-Körperteiles erzeugt wird und eine längliche Grundform aufweist, die mindestens an einem Ende offen ausgebildet ist und deren Innenform der Innenfläche des Körper-Anschlußstücks der herzustellenden Prothese entspricht, dadurch gekennzeichnet, daß eine je einem Ende zugeordnete Führungskappe (14,16) an dem Negativmodell (10) vorgesehen ist, die an dem ihr zugeordneten Ende befestigt ist und die je eine Lagervorrichtung (18,20) für eine Führungseinrichtung (12) aufweist, die sich von einer Führungskappe zu derjenigen am gegenüberliegenden Ende erstreckt und daß eine Abtastvorrichtung (22) verschiebbar und um die Mittelachse der Führungseinrichtung (12) drehbar gelagert ist und Meßdaten über das Profil des Negativmodells (10) abgibt, wobei die Prothese unter Verwendung dieser Meßdaten hergestellt wird.

2. Prothese nach Anspruch 1, dadurch gekennzeichnet, daß ein geschlossenes Ende des Negativmodells (10) aufgeschnitten wird.

3. Prothese nach Anspruch 1 oder 2, dadurch gekennzeichnet, daß die Führungseinrichtung als in elastischen Führungskappen (14,16) geführte Spindel (12) ausgebildet ist und daß die Abtastvorrichtung (22) gegenüber der Spindel (12) zugleich in Längsund in Umfangsrichtung bewegbar ist.

4. Prothese, die unter Verwendung eines Negativmodells hergestellt wird, wobei das Negativmodell durch Abformen des entsprechenden Patienten-Körperteiles erzeugt wird und eine längliche Grundform aufweist, die mindestens an einem Ende offen ausgebildet ist und deren Innenform der Innenfläche des Körper-Anschlußstücks der herzustellenden Prothese entspricht, dadurch gekennzeichnet, daß eine in den Innenraum des Negativmodells (10) einfahrbare Laserstrahlen aussendende Abtastvorrichtung mit einem Abtastkopf (105) vorgesehen ist, die verschiebbar und um die Längsmittelachse drehbar gelagert ist, wobei die Prothese unter Verwendung von Meßdaten hergestellt wird.

5. Prothese nach einem der vorhergehenden Ansprüche 1 bis 4, dadurch gekennzeichnet, daß die Abtastvorrichtung (22) und der Abtastkopf (105) zugleich einen Laserstrahl aussenden, deren Reflexionssignale an der Innenfläche des Negativmodells (10) erfaßbar sind und daß ein Analog/Digital-Wandler (38) vorgesehen ist, der ansprechend auf das insbesondere verstärkte Ausgangssignal der Abtastvorrichtung (22) dem Innenprofil des Negativmodells (10) entsprechende Meßdaten abgibt.

6. Prothese nach einem der vorangegangen Ansprüche 1 bis 5, dadurch gekennzeichnet, daß die Abtastvorrichtung (22;105) als Entfernungs-Abtastvorrichtung ausgebildet ist.

7. Prothese nach einem der vorhergehenden Ansprüche 1 bis 5, dadurch gekennzeichnet, daß die Abtastvorrichtung (22) als Entfernungs-Abtastvorrichtung ausgebildet ist und eine Antriebsvorrichtung (44) aufweist, mit welcher sie entlang eines im wesentlichen spiralförmigen Wegs antreibbar ist, wobei die Steigung etwa 2 mm pro Umdrehung beträgt.

8. Prothese nach einem der vorhergehenden Ansprüche 1 bis 5 und 7, dadurch gekennzeichnet, daß die Abtastvorrichtung (22) mit etwa 50 Umdrehungen pro Minute um die Führungseinrichtung (12) umläuft und dabei 200 bis 500, insbesondere 360, Meßpunkte pro Umdrehung aufnimmt.

9. Vorrichtung zur Herstellung einer Prothese, wie Oberschenkelprothese u.dgl., bei der ein Negativmodell unter Abformung von Restkörperteilen eines Patienten erstellt wird, wobei eine erhärtende Masse, insbesondere Gips, in verformbarem Zustand auf das Restkörperteil in einer Länge aufgebracht wird, die dem beabsichtigten Anlage-und Abstützbereich der Prothese an dem Restkörperteil entspricht, wobei das Negativmodell nach dem Erhärten in einer im wesentlichen kuppenförmigen Grundform von dem Restkörperteil abgenommen wird, dadurch gekennzeichnet, daß die Vorrichtung (100) aus einem kastenförmigen Geräteteil (101) mit einer in dessen Innenraum angeordneten Laserstrahlerzeugungseinrichtung und mit einer auf dessen oberen Abdeckplatte (108) angeordneten, ringförmigen Manschette (103) aus federnd-elastischen Werkstoffen, wie z.B. Gummi oder gummielastischen Kunststoffen, zur Herstellung und Aufnahme des mit seiner einseitigen Öffnung der Manschette zugekehrt aufgesetzten Negativmodells (10) besteht, wobei das Geräteteil (100) einen höhenverfahrbaren, in den Innenraum des Negativmodells einfahrbaren oder aus dessen Innenraum herausfahrbaren, in einem Winkel von etwa 45° Laserstrahlen aussendenden Abtastkopf (105) einer Abtastvorrichtung aufweist.

10. Vorrichtung nach Anspruch 9, dadurch gekennzeichnet, daß die Abtastvorrichtung (22) zugleich einen Laserstrahl aussendet, dessen Reflexionssignal an der Innenfläche des Negativmodells (10) erfaßbar ist und daß ein Analog/Digital-Wandler (38) vorgesehen ist, der ansprechend auf das insbesondere verstärkte Ausgangssignal der Abtastvorrichtung (22) dem Innenprofil des Negativmodells (10) entsprechende Meßdaten abgibt.

11. Vorrichtung nach Anspruch 9 und 10, dadurch gekennzeichnet, daß die Abtastvorrichtung (22) als Entfernungs-Abtastvorrichtung ausgebildet ist und der Abtastkopf (105) mit einer Antriebsvorrichtung (44) zusammenwirkt, mit welcher der Abtastkopf kontinuierlich angehoben und/oder abgesenkt wird.

12. Vorrichtung nach Anspruch 9 bis 11, dadurch gekennzeichnet, daß die Abtastvorrichtung (105) mit etwa 50 Umdrehungen pro Minute umläuft und dabei 200 bis 500, insbesondere 360, Meßpunkte pro Umdrehung aufnimmt.

13. Verfahren zur Herstellung einer Prothese, bei welchem ein Negativmodell unter Abformung von Restkörperteilen eines Patienten erstellt wird, wobei eine erhärtende Masse, insbesondere Gips, in verformbarem Zustand auf das Restkörperteil in einer Länge aufgebracht wird, die dem beabsichtigten Anlage-und Abstützbereich der Prothese an dem Restkörperteil entspricht, wobei das Negativmodell nach dem Erhärten in einer im wesentlichen kuppenförmigen Grundform von dem Restkörperteil abgenommen wird, dadurch gekennzeichnet, daß die Spitze des Negativmodells mit der kuppenförmigen Grundform abgeschnitten wird, so daß das Negativmodell eine im wesentlichen röhrenförmige Grundform erhält , daß auf jedes Ende der röhrenförmigen Grundform des Negativmodells eine Führungskappe aufgesetzt wird, die eine Lagervorrichtung aufweist, in welcher eine Führungseinrichtung gelagert ist, die sich zwischen den gegenüberliegenden Führungskappen erstreckt, daß die Führungskappen beim Aufsetzen auf die offenen Enden des Negativmodells zueinander so ausgerichtet werden, daß die Mittelachsen ihrer Lagervorrichtungen zueinander im wesentlichen koaxial sind, daß auf der Führungseinrichtung eine Abtastvorrichtung in Längsrichtung und in Umfangsrichtung der Führungseinrichtung geführt wird und daß die Abtastvorrichtung das Innenprofil des Negativmodells abtastet und die so ermittelten Meßwerte für die Erzeugung der Prothese mittels einer automatischen Einrichtung verwendet werden.

14. Verfahren nach Anspruch 13, dadurch gekennzeichnet, daß die Führungseinrichtung als Spindel ausgebildet ist, auf der die Abtastvorrichtung lösbar befestigt wird, und daß eine Antriebsvorrichtung die Spindel antreibt, so daß die Abtastvorrichtung über die Spindel entlang eines spiraligen Weges, insbesondere von unten nach oben, bewegt wird.

15. Verfahren nach einem der Ansprüche 13 oder 14, dadurch gekennzeichnet, daß die Führungseinrichtung an einer der Führungskappen elastisch gelagert wird, so daß Ausrichtfehler der Lagereinrichtungen zueinander tolerierbar sind.

16. Verfahren zur Herstellung einer Prothese, bei welchem ein Negativmodell unter Abformung von Restkörperteilen eines Patienten erstellt wird, wobei eine erhärtende Masse, insbesondere Gips, in verformbarem Zustand auf das Restkörperteil in einer Länge aufgebracht wird, die dem beabsichtigten Anlage-und Abstützbereich der Prothese an dem Restkörperteil entspricht, wobei das Negativmodell nach dem Erhärten in einer im wesentlichen kuppenförmigen Grundform von dem Restkörperteil abgenommen wird, dadurch gekennzeichnet, daß als Abtastvorrichtung ein in den Innenraum des Negativmodells mittels eines Teleskoptragarmes einfahrbarer und in der Höhe verfahrbarer Laserstrahlen aussendender Abtastkopf verwendet wird, der das Innenprofil des Negativmodells abtastet und die so ermittelten Meßwerte für die Erzeugung der Prothese mittels einer automatischen Einrichtung verwendet oder abrufbar gespeichert werden.

17. Verfahren nach einem der vorangegangenen Ansprüche 13 bis 16, dadurch gekennzeichnet, daß die von der Abtastvorrichtung abgegebenen Meßdaten in CNC-Steuerimpulse umgewandelt werden, die zur Steuerung einer CNC-Werkzeugmaschine zur Herstellung der Prothese dienen.

18. Verfahren nach einem der vorangegangenen Ansprüche 13 bis 17, dadurch gekennzeichnet, daß für die Erstellung der Negativmodelle vorgeformte Modellsegmente in Standardgrößen verwendet werden, die in die erhärtende Masse eingebettet werden.

19. Verfahren nach einem der vorangegangenen Ansprüche 13 bis 18, dadurch gekennzeichnet, daß die Spindel der Abtastvorrichtung oder der Teleskoptragarm für den Abtastkopf für die Übermittlung mindestens der Stromversorgungssignale für die Abtastvorrichtung verwendet wird.

# FIG.1

# FIG.3

# FIG.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 129 531 (N.V. STEWAL SA) <br> * Zusammenfassung; Seite 7, Zeilen 1-15; Abbildung 8 * | 1,5 | A 61 F 2/80 |
| | --- | | |
| A | IEEE TRANSACTIONS ON BIOMEDICAL ENGINEERING, Band BME-32, Nr. 4, April 1985, Seiten 257-262, IEEE, New York, US; D. DEAN et al.: "A software package for design and manufacture of prosthetic sockets for transtibial amputees" <br> * Seite 257 - Seite 259, Zeile 18 * | 1,5 | |
| | --- | | |
| A | BIOMEDIZINISCHE TECHNIK, Band 29, Nr. 12, Dezember 1984, Seiten 335-337, Berlin, DE; G. FERNIE: "Computer aided design and manufacture for prosthetics and orthotics" | 1 | RECHERCHIERTE SACHGEBIETE (Int. Cl.4) <br><br> A 61 F <br> A 61 C <br> A 61 B |
| | --- | | |
| A | EP-A-0 146 491 (MECRON) <br> * Seite 10, Zeile 11 - Seite 11, Zeile 6 * | 1 | B 23 Q <br> G 01 B |
| | --- | | |
| A | EP-A-0 121 617 (ARMCO) <br> * Seite 11, Zeile 12 - Seite 12, Zeile 14; Abbildungen 1-4 * | 1,4 | |
| | ---     -/- | | |

Der vorliegende Recherchenbericht wurde für alle Patentansprüche erstellt.

| Recherchenort | Abschlußdatum der Recherche | Prüfer |
|---|---|---|
| DEN HAAG | 21-04-1987 | WOLF C.H.S. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches
Patentamt

**EUROPÄISCHER RECHERCHENBERICHT**

Nummer der Anmeldung

EP 86 11 1347

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | GB-A- 926 506 (ASSOCIATED ELECTRICAL INDUSTRIES) * Seite 2, Zeilen 44-84; Abbildungen 2-4 * | 1,3 | |
| | --- | | |
| A | JP-A-60 138 407 (SUMITOMO KINZOKU) * Zusammenfassung * | 1,4,16 | |
| | --- | | |
| A | US-A-3 538 611 (D.E. NOWELL) * Abbildung 1 * | 1,4,16 | |
| | --- | | |
| A | FR-A-2 162 020 (L. HÄGGLUND) * Seite 6, Zeilen 23-35; Abbildung 7 * | 1 | |
| | ----- | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 21-04-1987 | WOLF C.H.S. |